# EUROPÄISCHE PATENTSCHRIFT

(11) **EP 3 443 883 B1**
(45) Veröffentlichungstag und Bekanntmachung des Hinweises auf die Patenterteilung: **29.07.2020**
(21) Anmeldenummer: 17186214.7
(22) Anmeldetag: 14.08.2017
(51) Int. Cl.: A61B 3/032

(54) **VORRICHTUNGEN UND VERFAHREN ZUR DURCHFÜHRUNG AUGENBEZOGENER MESSUNGEN**
METHOD AND DEVICES FOR PERFORMING EYE-RELATED MEASUREMENTS
PROCÉDÉS ET DISPOSITIF PERMETTANT D'EFFECTUER DES MESURES RELATIVES À L' OEIL

(43) Veröffentlichungstag der Anmeldung: 20.02.2019
(73) Patentinhaber: Carl Zeiss Vision International GmbH, 73430 Aalen (DE)
(72) Erfinder: BREUNINGER, Tobias, 89542 Herbrechtingen (DE); SEESSELBERG, Markus, 73431 Aalen (DE); KUBITZA, Matthias, 73434 Aalen (DE); WIDULLE, Frank, 89233 Neu-Ulm (DE)
(74) Vertreter: Sticht, Andreas

(56) Entgegenhaltungen:
- JP-A- 2003 029 198
- US-A1- 2013 201 446

## Beschreibung

Die vorliegende Anmeldung betrifft Vorrichtungen und Verfahren zur Durchführung augenbezogener Messungen.

Unter augenbezogenen Messungen sind dabei allgemein Messungen zu verstehen, welche Eigenschaften von Augen einer Person für sich genommen ermitteln oder einen anderen Gegenstand, insbesondere ein Brillenglas, bezüglich der Augen einer Person vermessen. Ein Beispiel für eine Vorrichtung zur Messung von Eigenschaften der Augen selbst ist ein Refraktometer zur objektiven Refraktionsmessung, mit welchem die Refraktion von Augen einer zu untersuchenden Position bestimmt wird und üblicherweise in Form von Sphäre, Zylinder und Achse wie in der DIN ISO 13666: 2012 definiert ausgegeben wird. Ein anderes Beispiel ist ein Perimeter, mit welchem ein Gesichtsfeld einer Person bestimmt wird (vgl. Wikipedia-Artikel "Perimetrie", Stand 17. Juli 2017). Ein weiteres Beispiel ist eine Vorrichtung zur Erzeugung von Schnittbildern der Vorderkammer oder der Retina eines Auges, beispielsweise mittels optischer Kohärenztomographie (OCT), siehe Wikipedia-Artikel "Optische Kohärenztomographie", Stand 17. Juli 2017. Weitere Beispiele umfassen Vorrichtungen zur Biometrie des Auges, welche die Auswahl einer patientenspezifischen Intraokularlinse vor einer Kataraktoperation erlauben, d.h. die Auswahl einer in das Auge einzusetzenden Linse. Ein solches Gerät wird von der Carl Zeiss Gruppe unter der Bezeichnung IOL Master vertrieben. Mittels derartiger augenbezogenen Messungen, die Eigenschaften des Auges selbst bestimmen, werden also Messdaten (z.B. die oben erwähnte Refraktion) gewonnen, die einen Zustand des Auges beschreiben. Auf Basis dieser Messdaten kann ein Arzt oder Optiker dann eine Diagnose stellen, indem er überprüft, ob die Messdaten ein Krankheitsbild anzeigen. Im Falle der Refraktion kann die Diagnose dann z.B. auf Kurzsichtigkeit oder Weitsichtigkeit lauten. Diese Erstellung der Diagnose ist also ein weiterer Vorgang nach der Bereitstellung der Messdaten durch die augenbezogene Messung.

Ein Beispiel für eine Vorrichtung, die ein Brillenglas bezogen auf ein Auge zum Zwecke der Positionierung des Brillenglases vermisst, sind sogenannte Zentriervorrichtungen. Zentriervorrichtungen sind in der WO 2005/069063 A oder in den europäischen Patentanmeldungen Nr. 17 153 559.4 und 17 153 556.0 beschrieben. Zentriervorrichtungen bestimmen sogenannte Zentrierparameter, welche von einem Optiker für ein korrektes Einschleifen und Anpassen des Brillenglases benutzt werden, wie in diesen Dokumenten näher erläutert.

Einen Überblick über verschiedene Geräte zur Refraktion und Zentriermessung gibt auch die Seite https://www.zeiss.de/vision-care/de_de/products-services/instrumentesysteme/sehanalyse-und-refraktion.html, Stand 17. Juli 2017. Für die oben genannten Vorrichtungen ist eine Vielzahl von weiteren Beispielen im Stand der Technik bekannt. Daher werden diese hier nicht näher erläutert.

Bei derartigen Vorrichtungen ist es zur Messung häufig erforderlich, dass die zu untersuchende Person eine bestimmte Blickrichtung einnimmt. Beispielsweise wird bei Zentriermessungen häufig davon ausgegangen, dass die Person den Blick horizontal im Wesentlichen ins Unendliche gerichtet hat.

Um zu gewährleisten, dass die Person eine für die jeweilige Messung erforderliche Blickrichtung einnimmt, wird ein Fixationstarget bereitgestellt. Unter einem Fixationstarget wird im Rahmen der vorliegenden Anmeldung ein reales oder virtuelles Objekt oder Bild verstanden, auf welches die Person ihren Blick bei der jeweiligen Messung richten soll. Virtuell ist das Objekt oder Bild dabei dann, wenn es sich nicht um ein tatsächliches Bild oder Objekt handelt, sondern beispielsweise um ein in den Raum projiziertes Bild oder ein Spiegelbild.

Viele Vorrichtungen, z.B. die unter www.rodenstock.de/de/brillenglaeser/rodenstocktechnologien/3D-videovermessung.html, Stand 17. Juli 2017, gezeigte Vorrichtung zur 3D-Videovermessung arbeitet so, dass die zu untersuchende Person aus einem relativ kurzen Abstand, z.B. 1 m, ein Objekt, das Spiegelbild der Person selber oder eine an der Vorrichtung angebrachte Leuchtdiode fixiert. Eine Videokamera nimmt dann Bilder der Person auf, während diese das so gebildete Fixationstarget betrachtet. Nachteilig hieran ist, dass, wenn die Person ihren Blick auf das Fixationstarget einnimmt, die Augen eine sogenannte Konvergenzstellung einnehmen, d.h. nicht ins Unendliche gerichtet sind, sondern die Blickrichtung beider Augen auf den Punkt des Fixationstargets, welches sich relativ nahe an der Person befindet (beispielsweise in dem erwähnten Abstand von 1 m) zuläuft. Die rein geometrische Konvergenz, die sich aus der Position der Augen und des Fixationstargets ergibt, kann dabei rechnerisch kompensiert werden. Wie in der WO 2005/069063 A1 erläutert entspricht diese geometrische Konvergenz jedoch nicht notwendigerweise der tatsächlichen Konvergenz des Augenpaares, d.h. die tatsächliche Stellung der Augen beim Betrachten des Fixationstargets kann von der aus rein geometrischen Überlegungen ermittelten Stellung abweichen. Dies kann wiederum zu Ungenauigkeiten bei der Messung führen.

Daher schlägt die WO 2005/069063 A1 eine Vorrichtung zur Erzeugung eines Fixationstargets vor, bei welcher mittels einer Laserlichtquelle durch ein diffraktives Element ein ins Unendliche projiziertes Specklemuster erzeugt wird. Hierzu wird bei der Vorrichtung der WO 2005/069063 A1 das Laserlicht über ein diffraktives Element auf einen Schirm projiziert, um auf dem Schirm ein Specklemuster zu bilden, und dieses Muster wird dann mit einem optischen System ins Unendliche projiziert. Beispielsweise kann das optische System eine Lupenabbildung mit einer Linse oder Linsengruppe realisieren. Ein diffraktives Element ist dabei ein Element, das auf Basis von Lichtbeugung arbeitet, im Gegensatz zu refraktiven Elementen wie Linsen, welche auf Basis von Lichtbrechung arbeiten. Das Specklemuster kann mit einem zusätzlichen Muster, beispielsweise einem kreuzförmigen Muster, überlagert werden, wie ebenfalls in der WO 2005/069063 A1 erläutert.

Indem das Specklemuster ins Unendliche projiziert wird, nimmt die Person grundsätzlich eine Blickrichtung ein, bei welcher der Blick ins Unendliche gerichtet ist. Daher muss hier keine Kompensation aufgrund einer Konvergenz der Augen auf einen vergleichsweise nahe liegenden Punkt durchgeführt werden.

Dennoch weist das Fixationstarget der WO 2005/069063 A1 noch Nachteile auf. Zum einen gibt es Personen, die aufgrund der subjektiven Nähe der Vorrichtung eine Art Restkonvergenz aufzeigen, d.h. die Augen haben nicht genau die Blickrichtung unendlich.

Zusätzlich wird bei der Vorrichtung gemäß diesem Stand der Technik das Bild mit beiden Augen fixiert (binokulares Sehen), wobei der typische Abstand zwischen den Augen etwa 64 mm beträgt. Daher wird das optische System, mit welchem das Fixationstarget auf Basis des Laserlichts erzeugt wird, außerhalb seiner optischen Achse verwendet. Die optische Achse ist dabei bei einem rotationssymmetrischen optischen System die Symmetrieachse des Systems und geht insbesondere durch Krümmungsmittelpunkte gekrümmter Flächen hindurch. Optische Systeme weisen Abbildungsfehler wie sphärische Aberration oder Verzeichnung auf, die abseits der optischen Achse stärker ausfallen. Diese Abbildungsfehler können durch verschiedene Maßnahmen, beispielsweise die Verwendung asphärischer Linsen, reduziert werden, was jedoch die Kosten erhöht.

Zudem ist die Vorrichtung gemäß der WO 2005/069063 A1 relativ groß. Da bei dieser Vorrichtung ein klassisches optisches System mit refraktiven (brechenden) Elementen, insbesondere Linsen, verwendet wird, muss ein bestimmter Abstand zwischen einer verwendeten Linse und einem Schirm, auf welchen das Laserlicht als Specklemuster projiziert wird, eingehalten werden. Dieser Abstand entspricht etwa der Brennweite der Linse. Je kürzer die Brennweite des Systems ist, umso stärker werden Abbildungsfehler sichtbar. Größere Brennweiten vergrößern hingegen den benötigten Bauraum.

Schließlich ist die Vorrichtung gemäß der WO 2005/069063 A1 vergleichsweise teuer. Es werden bei der Vorrichtung der WO 2005/069063 A1 zur Erzeugung des Fixationstargets zumindest ein Laser, ein diffraktives Element zum Erzeugen eines zu projizierenden Musters (insbesondere Specklemuster), ein Schirm und eine Abbildungsoptik, bestehend typischerweise aus einer oder mehreren Linsen, verwendet. Jede dieser Komponenten kostet Geld.

Die US 2013/0201446 A1 offenbart ein Hologramm, welches ein oder mehrere holografische Bilder codiert. Die holografischen Bilder können als Sehtafeln oder zum Augentraining verwendet werden. Dabei können die holografischen Bilder im Wesentlichen im Unendlichen oder in einer endlichen Distanz erzeugt werden. Ein Umschalten zwischen den holografischen Bildern im Falle mehrerer holografischer Bilder ist dabei nicht erläutert. Zudem wird die Verwendung derartiger holografischer Bilder als Fixationstarget erwähnt.

Es ist ausgehend davon eine Aufgabe, eine Vorrichtung zur Durchführung einer augenbezogenen Messung mit einer Einrichtung zum Erzeugen eines Fixationstargets bereitzustellen, bei welcher eine Flexibilität hinsichtlich der Erzeugung verschiedener Fixationstargets erhöht ist.

Hierzu wird eine Vorrichtung zur Durchführung einer augenbezogenen Messung nach Anspruch 1 sowie ein Verfahren nach Anspruch 10 bereitgestellt. Die Unteransprüche definieren weitere Ausführungsformen.

Erfindungsgemäß wird eine Vorrichtung zur Durchführung einer augenbezogenen Messung bereitgestellt, welche eine Einrichtung zur Erzeugung eines Fixationstargets sowie eine Einrichtung zur Durchführung der augenbezogenen Messung, wenn eine Person auf das Fixationstarget blickt, umfasst. Die Vorrichtung ist dadurch gekennzeichnet, dass die Einrichtung zur Erzeugung des Fixationstargets ein holografisches Element umfasst.

Die Vorrichtung umfasst weiter eine Beleuchtungseinrichtung zum Beleuchten des holografischen Elements.

Die Vorrichtung umfasst weiter eine Beleuchtungseinrichtung zum Beleuchten des holografischen Elements.

Die Einrichtung zur Durchführung der augenbezogenen Messung kann dabei irgendeine für sich genommen bekannte Einrichtung sein, beispielsweise eine Zentriereinrichtung, ein

Refraktometer, ein Perimeter, eine Einrichtung zur Erzeugung von Schnittbildern der Vorderkammer oder der Retina oder eine Einrichtung zur Biometrie des Auges, wie sie eingangs erläutert wurden. Da wie eingangs erläutert derartige Einrichtungen für sich genommen in vielen Varianten bekannt sind und sich die vorliegende Erfindung insbesondere auf die Ausgestaltung der Einrichtung zur Erzeugung des Fixationstargets bezieht, wird die Einrichtung zur Durchführung der augenbezogenen Messung selbst hier nicht näher erläutert.

Umfasst die Einrichtung zur Erzeugung des Fixationstargets ein holografisches Element und eine Beleuchtungseinrichtung, so kann die Beleuchtungseinrichtung eine oder mehrere Lichtquellen umfassen, um das holografische Element zu beleuchten. Als Lichtquelle kann insbesondere eine Laserlichtquelle wie eine Laserdiode dienen, wobei beispielsweise bei Weißlichthologrammen auch andere Arten von Lichtquellen verwendbar sind.

Ein holografisches Element ist eine Komponente, die ein oder mehrere Hologramme umfasst. Ein Hologramm ist dabei eine Art Bild, welches im Gegensatz zu einer normalen Fotografie nicht nur die Intensität einfallenden Lichtes aufzeichnet, sondern Intensität und die Phase. Die Bildaufnahme geschieht dabei mit Hilfe der Interferenz, wozu kohärentes Licht, in der Regel ein Laserstrahl, verwendet wird, der beispielsweise mittels Streulinsen aufgeweitet wird. Dabei wird auf einem lichtempfindlichen Material ein Referenzstrahl mit einem Beleuchtungsstrahl, welcher ein Objekt, das auf dem Hologramm aufgenommen wird, beleuchtet, zur Interferenz gebracht. Das so belichtete lichtempfindliche Material kann nach einem Entwicklungsschritt entweder direkt als Hologramm dienen oder zur Herstellung von entsprechenden Hologrammen mittels Replikationsverfahren verwendet werden. Auf diese Weise lässt sich eine große Anzahl gleichartiger Hologramme kostengünstig herstellen. Wird dann das Hologramm mit entsprechendem kohärentem Licht aus derjenigen Richtung, aus der bei der Bildaufnahme der Referenzstrahl auf das lichtempfindliche Material fiel, beleuchtet, erscheint das Objekt an der Stelle, an der sich das Objekt bei der Bildaufnahme befand. Bei einer anderen Art von Hologrammen, auch als holografische Mattscheibe bezeichnet, werden zwei Lichtstrahlen zur Interferenz gebracht, wobei in diesem Fall in dem Beleuchtungsstrahl kein Objekt vorhanden ist, sondern der Beleuchtungsstrahl von einem Punkt ausgeht. So kann eine holografische Mattscheibe erzeugt werden, bei welcher das Hologramm dann aus Richtung der Referenzwelle mit einem Laser abgerastert werden kann und etwa an der Stelle des Punktes des Beleuchtungsstrahls dann ein Objekt entsteht. Die Form des Objekts wird durch ein Ein- und Ausschalten des Laserlichts während dem Abrastern definiert. Dieses Prinzip wird beispielsweise auch für die Dateneinspiegelung verwendet, wie in der DE 10 2015 101 687 A1 beschrieben.

Näheres zur Herstellung von Hologrammen findet sich beispielsweise im Wikipedia-Artikel "Holografie", Stand 17. Juli 2017. Wie in dem oben genannten Artikel erläutert wird, lassen sich Hologramme nach verschiedenen Eigenschaften klassifizieren, insbesondere in Volumen- und Flächenhologramme sowie in Amplituden- und Phasenhologramme. Bei Volumenhologrammen sind die holografischen Informationen (d.h. ein Interferenzmuster) auch in einer Dickenrichtung des Hologramms gespeichert, während bei Flächenhologrammen die Interferenz im Wesentlichen in einer Ebene lichtempfindlichen Materials aufgezeichnet wird. Zudem kann zwischen Weißlichthologrammen und Hologrammen, die nicht unter weißem Licht rekonstruiert werden können, sowie echtfarbigen Hologrammen unterschieden werden, wobei nur Volumenhologramme Weißlichthologramme sein können. Das Hologramm kann als Transmissionshologramm oder als Reflexionshologramm ausgestaltet sein. Bei einem Transmissionshologramm wird das Hologramm von einer Seite beleuchtet und von der anderen Seite betrachtet. Ein virtuelles holografisches Objekt entsteht dabei auf der gleichen Seite des Hologramms, von der auch beleuchtet wird. In diesem Fall ist das Hologramm also zwischen der Lichtquelle der Beleuchtungseinrichtung und dem Betrachter angeordnet. Bei einem Reflexionshologramm erfolgt die Beleuchtung durch die Lichtquelle von der gleichen Seite, von der auch der Betrachter das Hologramm ansieht. Diese Hologrammtypen sind ebenfalls in dem obigen Wikipedia-Artikel "Holografie" erläutert. Als Hologramm des holografischen Elements kann grundsätzlich jeder der Hologrammtypen verwendet werden, wobei Volumenhologramme bevorzugt sind. Diese lassen sich kostengünstig herstellen und können in Dickenrichtung, d.h. einer Richtung senkrecht zur Oberfläche, auch mehrere Hologramme übereinander gestapelt enthalten.

Wenn das holografische Element mittels der Beleuchtungseinrichtung oder durch eine Fremdlichtquelle beleuchtet wird, erzeugt es gemäß den obigen Erläuterungen das Fixationstarget als holografisches Bild. Dieses holografische Bild wird im Rahmen der vorliegenden Anmeldung auch als virtuelles holografisches Objekt bezeichnet. Virtuell deswegen, weil es sich nicht um ein reales Objekt handelt, sondern eben um ein Bild eines bei der Herstellung des Hologramms wie eingangs erläutert verwendeten Objekts oder- im Falle einer holografischen Mattscheibe als holografisches Element - als Bild eines mittels der Lichtquelle auf das holografische Element beleuchteten Musters.

Durch die Beleuchtung mit der Lichtquelle erzeugt das Hologramm dann das Fixationstarget an einer Stelle, welche vom Design bei der Aufnahme des Hologramms wie oben erläutert abhängt, d.h. an der Stelle, an der bei der Aufnahme das Objekt platziert wurde. Eine Realisierung als holografische Mattscheibe wie oben erläutert ist ebenso möglich. In diesem Fall kann die Form eines projizierten Objekts durch entsprechendes Abrastern und Ansteuern der Lichtquelle realisiert werden, wie in der DE 10 2015 101 687 A1 für eine andere Anwendung, nämlich die Dateneinspiegelung, erläutert.

Die Verwendung eines holografischen Elements zur Erzeugung des Fixationstargets weist folgende Vorteile gegenüber dem Stand der Technik auf:
Mittels des holografischen Elements kann bei geringen Herstellungskosten eine sehr gute optische Qualität erreicht werden. Das durch das holografische Element erzeugte virtuelle Bild des Objekts kann gut aus verschiedenen Richtungen betrachtet werden. Dadurch ist ein solches Fixationstarget speziell für die binokulare Verwendung, d.h. zur Betrachtung mit beiden Augen, sehr gut geeignet. Zudem wird das so erzeugte holografische Fixationstarget subjektiv von der Person als ein im Raum schwebendes reales Fixationsobjekt wahrgenommen. Damit wird der Nachteil einer möglichen Restkonvergenz eliminiert. Des Weiteren kann das Hologramm mit einer kleinen Laserlichtquelle unter einem relativ steilen Winkel beleuchtet werden (wenn bei der Herstellung der Referenzstrahl unter einem entsprechenden steilen Winkel auf das lichtempfindliche Material gelenkt wird, wie oben beschrieben). So kann ein kleinerer Bauraum als im Stand der Technik realisiert werden. Schließlich können durch das holografische Element verglichen mit dem erläuterten Stand der Technik das diffraktive Element, der Schirm und das optische System ersetzt werden. Hierdurch ist eine günstigere Herstellung möglich als bei der Einrichtung der WO 2005/069063 A1.

Bevorzugt ist das holografische Element eingerichtet, das Fixationstarget bei Beleuchtung in einer Entfernung von mindestens 4 m, bevorzugt mindestens 8 m, entfernt von dem holografischen Element zu erzeugen. Bei derartigen Entfernungen entspricht die Konvergenz der Augen bei Betrachtung des Fixationstargets zumindest näherungsweise einem Blick ins Unendliche. Derartige Abstände lassen sich einfach realisieren, indem beim Beleuchten ein Objekt, welches von dem Beleuchtungsstrahl beleuchtet wird, in einem entsprechenden Abstand von einem bei der Herstellung des Hologramms verwendeten lichtempfindlichen Material angeordnet ist.

Das Fixationstarget weist dabei bevorzugt einen zu fixierenden Bereich, d.h. einen Bereich, den die Person bei einer augenbezogenen Messung fixieren soll, mit einer Ausdehnung auf, welche einem Betrachtungswinkel <1°, bevorzugt <0,5° gesehen von dem holografischen Element aus entspricht. Dieser Winkel entspricht im Wesentlichen dem Arkustangens der Ausdehnung des zu fixierenden Bereichs des Fixationstargets geteilt durch den Abstand des Fixationstargets vom holografischen Element. Die Ausdehnung ist dabei senkrecht zu dem Abstand zu verstehen. Bei einem Abstand von 8 m ergibt sich z.B. eine Ausdehnung von ca. kleiner 14 cm, bevorzugt kleiner ca. 7 cm. Mit einer derart kleinen Ausdehnung kann eine definierte Blickrichtung der Augen erzielt werden. Bei größeren Ausdehnungen könnte es hier zu Ungenauigkeiten kommen, da die Person verschiedene Teile eines ausgedehnten Fixationstargets betrachten könnte, was zu entsprechenden unterschiedlichen Augenstellungen führen würde. Bei dem zu fixierenden Bereich handelt es sich dabei um einen von anderen Bereichen des Fixationstargets unterscheidbaren Bereich, sodass der Person die Anweisung gegeben werden kann, diesen Bereich zu fixieren. Das gesamte Fixationstarget kann dabei eine größere Ausdehnung (z.B. entsprechend einem Betrachtungswinkel von 20° oder mehr) aufweisen, wobei dieser Winkel im Wesentlichen dem Arkustanges der Abmessung des gesamten Fixationstargets geteilt durch den Abstand des Fixationstargets von dem holografischen Element ist, um es stark fehlsichtigen Personen zu erleichtern, das Fixationstarget überhaupt zu erkennen.

Hologramme, insbesondere Volumenhologramme, sind wellenlängenselektiv und winkelselektiv. Wellenlängenselektiv bedeutet, dass das Hologramm nur bei Beleuchtung mit derjenigen Wellenlänge, mit welcher es beleuchtet wurde, auch ein Bild erzeugt (innerhalb eines gewissen Toleranzbereichs). Winkelselektiv bedeutet, dass das Hologramm nur bei Beleuchtung unter demjenigen Winkel (wiederum mit einer gewissen Toleranz), unter dem es mit dem Referenzstrahl beleuchtet wurde, ein Bild erzeugt. Diese Eigenschaften lassen sich bei bevorzugten Ausführungsformen für das wahlweise Beleuchten ausnutzen, um die erfindungsgemäße Einrichtung zum Erzeugen des Fixationstargets mit zusätzlichen Merkmalen auszustatten.

Bei einem Ausführungsbeispiel kann die Einrichtung zum Erzeugen des Fixationstargets in verschiedenen Farben eingerichtet sein. In diesem Fall umfasst das holografische Element zwei oder mehr Hologramme, welche jeweils mit kohärentem Licht unterschiedlicher Wellenlänge erzeugt wurden. Im Falle von Volumenhologrammen können diese zwei oder mehr Hologramme in einer Dickenrichtung des Hologramms übereinander angebracht werden. Die Dickenrichtung ist dabei eine Richtung senkrecht zu einer Oberfläche des Volumenhologramms, insbesondere einer Oberfläche, die zum Erzeugen des Fixationstargets beleuchtet wird. Es ist aber ebenso eine Bereitstellung nebeneinander oder in separaten Hologrammen möglich. Entsprechend umfasst die Beleuchtungseinrichtung dann mehrere Lichtquellen der entsprechenden verschiedenen Wellenlängen (einschließlich der bereits erwähnten Lichtquelle) zum Erzeugen des ersten und des zweiten Fixationstargets mit verschiedenen Farben.

Mittels dieser verschiedenen Farben können dann einer Person, die von der Vorrichtung untersucht wird, Informationen gegeben werden. Unter Informationen sind dabei Hinweise, Anweisungen und/oder Rückmeldungen an die Person zu verstehen, zusätzlich zum bloßen Vorhandensein eines Fixationstargets.

Wie bei der in den europäischen Patentanmeldungen Nr. 17 153 559.4 und 17 153 556.0 beschriebenen Zentriervorrichtung kann die Position des Kopfes der Person mittels Kameras erfasst werden und mit einer Soll-Position für die Zentriermessung verglichen werden. Über die Farbe des Fixationstargets kann der Person dann als Information eine Rückmeldung gegeben werden, ob die Position des Kopfes korrekt ist, z.B. anhand von Ampelfarben rot, gelb und grün.

Ebenso wie verschiedene Farben können alternativ oder zusätzlich auch verschiedene Formen wahlweise dargestellt werden. Hierzu werden die zwei oder mehr Hologramme mittels Objekten, die solche verschiedene Formen aufweisen, beleuchtet, oder es wird die oben erwähnte holografische Mattscheibe verwendet. Die verschiedenen Formen des ersten und zweiten Fixationstargets können beispielsweise Pfeile sein, die der Person als Information anzeigen, in welche Richtung sie ihren Kopf bewegen soll, um eine Soll-Position für die Messung zu erreichen. Indem das Fixationstarget selbst verschiedene Farben oder Formen annimmt, kann die Person die durch die Farben oder Formen kommunizierte Information erhalten, ohne den Blick von dem Fixationstarget abzuwenden.

Die Einrichtung zum Erzeugen des Fixationstargets kann zudem eingerichtet sein, das zweite Fixationstarget (oder mehrere Fixationstargets) an einer anderen Position als das erste Fixationstarget zu erzeugen. Hierzu können wie bei der Herangehensweise für verschiedene Farben oder Formen zwei oder mehr Hologramme verwendet werden. Diese können gleichzeitig kodiert sein, beispielsweise in verschiedenen Schichten des Volumenhologramms oder als entsprechendes computererzeugtes Hologramm (computer generated hologram, CGH, siehe Wikipedia-Artikel "Computer generated holography", Stand 27.7.2017 ausgestaltet sein. Sie können durch verschiedene Lichtquellen "geschaltet" werden, beispielsweise mit verschiedenen Lichtwellenlängen (Lichtwellenlängenselektivität von Hologrammen) oder durch Beleuchtungen unter verschiedenen Winkeln (Winkelselektivität von Hologrammen). Die Hologramme können auch örtlich getrennt in der holografischen Einrichtung angeordnet sein und auf diese Weise separat beleuchtet werden, so dass verschiedene Beleuchtungsorte verwendet werden. Erfindungsgemäß wird durch verschiedene Beleuchtungsarten (unterschiedlich hinsichtlich Lichtwellenlänge, Beleuchtungswinkel und/oder Beleuchtungsort) zwischen verschiedenen Farben, Formen und/oder Positionen für das Fixationstarget gewählt.

Neben verschiedenen Lichtquellen für verschiedene Beleuchtungsarten können unterschiedliche Beleuchtungswinkel oder Beleuchtungsorte auch mit einer einzigen Lichtquelle, die dann durch bewegliche Spiegel unter verschiedenen Winkeln und/oder an verschiedenen Orten auf das holografische Element gelenkt wird, realisiert werden.

Während in diesem Fall das erste Fixationstarget wie oben erläutert in einer großen Entfernung, >4 m oder >8 m, erzeugbar sein kann und somit als Fixationstarget für einen Blick in die Ferne (näherungsweise ins Unendliche) dient, kann das zweite Fixationstarget als sogenanntes Nahblickziel dienen, d.h. in kurzer Entfernung (beispielsweise <1 m, oder <50 cm, insbesondere ca. 30 cm) angeordnet sein, also im Bereich einer typischen Leseentfernung. Aus Messungen, wenn die Person den Blick dann auf dieses Nahblickziel richtet, kann auf eine Augenstellung beim Lesen rückgeschlossen werden, was für die sogenannte Nahzentrierung verwendet werden kann. Zudem kann aus derartigen Messungen der Augendrehpunkt ermittelt werden. Die Nahzentrierung dient zur Zentrierung bei Gleitsichtgläsern, welche einen Nahteil zum Sehen in der Nähe, insbesondere Lesen, aufweisen. Diese Nahzentrierung sowie die Bestimmung des Augendrehpunkts kann unter Verwendung des mit der erfindungsgemäßen Vorrichtung erzeugten Nahblickziels in für sich genommen bekannter Weise erfolgen, beispielsweise wie in der europäischen Patentanmeldung EP 17 174 925.2 beschrieben.

Hierdurch können auf einfache Weise und mit geringem Bauraum verschiedene Fixationstargets für verschiedene Zwecke bereitgestellt werden.

Alternativ zu dem oben beschriebenen wahlweisen Erzeugen von dem ersten Fixationstarget für den Blick in die Ferne und dem zweiten Fixationstarget als Nahblickziel in Abhängigkeit von der Beleuchtungsart kann auch ein einzigen Fixationstarget mit einem ersten Teil, der einen Abstand von mehr als 4 m zu dem holografischen Element aufweist und somit zum Fixieren beim Blick in die Ferne dienen kann, und einem zweiten Teil, der einen Abstand von weniger als 1 m zu dem holografischen Element aufweist und somit als Nahblickziel dienen kann, erzeugt werden. In diesem Fall weist das virtuelle holografische Objekt den ersten Teil und den zweiten Teil und somit eine entsprechende räumliche Ausdehnung auf. Diese Variante ist jedoch weniger bevorzugt, da beide Teile gleichzeitig für die zu untersuchende Person sichtbar sind und es deswegen passieren kann, dass die Person ihren Blick auf den für eine jeweilige augenbezogene Messung "falschen" Teil (z.B. auf den zweiten Teil für eine Fernzentrierung oder den ersten Teil für die Nahzentrierung) gerichtet ist oder der Blick der Person zwischen dem ersten und zweiten Teil hin- und herwandert.

Die Einrichtung zum Erzeugen des Fixationstargets kann weiter eine transparente Schutzabdeckung, beispielsweise eine Glasplatte, aufweisen, um die Einrichtung vor Schmutz oder Umwelteinflüssen zu schützen.

Gemäß einem weiteren Aspekt wird ein Verfahren bereitgestellt, umfassend:
Beleuchten eines holografischen Elements zum Erzeugen eines Fixationstargets, und Durchführen einer augenbezogenen Messung, während eine Person auf das Fixationstarget blickt.

Wie für die obige Vorrichtung erläutert kann mittels des holografischen Elements auf einfache Art und Weise ein Fixationstarget erzeugt werden. Die augenbezogene Messung kann wiederum jegliche Art von Messung sein, bei welcher eine Blickrichtung der Person durch das Fixationstarget festgelegt sein soll, wie die oben erläuterten Zentriermessungen, Refraktionsmessungen, Biometriemessungen und dergleichen.

Die oben erwähnten Varianten der Vorrichtung können in entsprechender Weise auch auf das Verfahren angewendet werden, mit den entsprechenden Vorteilen. So wird erfindungsgemäß das holografische Element wahlweise zum Erzeugen von Fixationstargets in verschiedenen Farben und/oder Formen beleuchtet, oder wahlweise zum Erzeugen von Fixationstargets an verschiedenen Orten (beispielsweise als Nahblickziel und Fernblickziel) beleuchtet. Hierzu kann die Beleuchtung hinsichtlich Lichtwellenlänge, Beleuchtungswinkel und/oder Beleuchtungsort variiert werden, wie beschrieben. Das Fixationstarget kann mit einer Ausdehnung eines zu fixierenden Bereichs erzeugt werden, sodass sich für die Person ein Betrachtungswinkel <1°, bevorzugt <0,5° ergibt.

Die Erfindung wird nachfolgend unter Bezugnahme auf die beigefügte Zeichnung anhand von Ausführungsbeispielen näher erläutert. Es zeigen:
Fig. 1 eine Vorrichtung zur Zentriermessung gemäß einem Ausführungsbeispiel,
Fig. 2 ein Ausführungsbeispiel einer Einrichtung zum Erzeugen eines Fixationstargets mit einem Transmissionshologramm,
Fig. 3 ein Ausführungsbeispiel einer Einrichtung zum Erzeugen eines Fixationstargets mit einem Reflexionshologramm,
Fig. 4 ein Ausführungsbeispiel einer Einrichtung zum Erzeugen eines Fixationstargets wahlweise an verschiedenen Orten,
Fig. 5 ein Flussdiagramm zur Veranschaulichung eines Verfahrens gemäß einem Ausführungsbeispiel, und
Fig. 6 ein Beispiel für ein Fixationstarget, wie es von einer erfindungsgemäßen Vorrichtung erzeugbar ist.

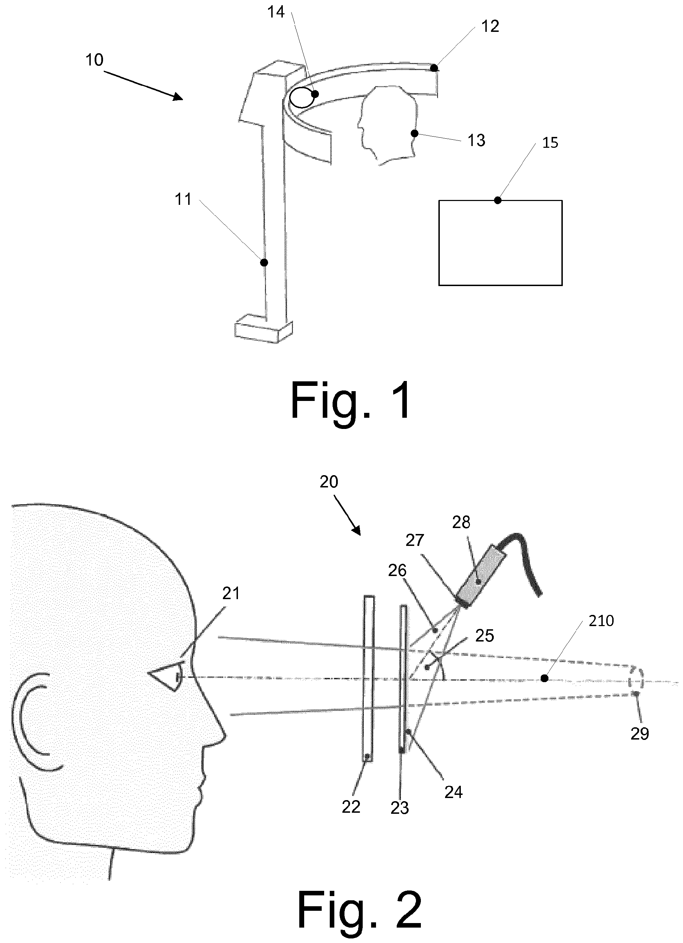

Die Fig. 1 zeigt ein Beispiel für eine Zentriervorrichtung gemäß einem Ausführungsbeispiel. Die Vorrichtung der Fig. 1 weist eine halbkreisförmige Anordnung von Kameras 12 auf, welche auf einer Säule 11 befestigt ist. Eine Person stellt sich dann derart hin, dass ein Kopf 13 der Person wie in der Fig. 1 gezeigt in der halbkreisförmigen Kameraanordnung 12 positioniert ist und aus verschiedenen Richtungen aufgenommen werden kann.

Die Vorrichtung 10 der Fig. 1 weist weiter eine Einrichtung 14 zum Erzeugen eines Fixationstargets auf, auf welches die Person während der Bildaufnahme ihren Blick richten soll. Die Einrichtung 14 umfasst dabei ein holografisches Element, um das Fixationstarget zu erzeugen. Ausführungsbeispiele für derartige Einrichtungen zum Erzeugen eines Fixationstargets werden später unter Bezugnahme auf die Figuren 2-4 näher erläutert.

Die aufgenommenen Bilder werden dann von einer Recheneinrichtung 15 ausgewertet, beispielsweise um Zentrierparameter bei der Zentriermessung zu bestimmen. Abgesehen von der Bereitstellung der Einrichtung 14, welche ein holografisches Element umfasst, entspricht die Vorrichtung der Fig. 1 der in den europäischen Patentanmeldungen Nummer 17 153 559.4 sowie 17 153 556.0 beschriebenen Vorrichtungen und werden daher nicht weiter erläutert. Insbesondere stellt die Vorrichtung 10 nur ein mögliches Beispiel für eine Vorrichtung zur Durchführung einer augenbezogenen Messung dar, in der die Einrichtung 14 zum Erzeugen des Fixationstargets verwendet werden kann, wie bereits eingangs erläutert.

Die Fig. 2 zeigt ein Ausführungsbeispiel einer Einrichtung 20 zum Erzeugen eines Fixationstargets 29 gemäß einem Ausführungsbeispiel. Diese Einrichtung 20 kann beispielsweise als Einrichtung 14 der Fig. 1 verwendet werden.

Die Einrichtung 20 der Fig. 2 umfasst ein Glassubstrat 23 mit einer holografischen Schicht 24, d.h. einer Schicht, in der ein oder mehrere Hologramme angeordnet sind. Des Weiteren umfasst die Einrichtung 20 der Fig. 2 eine Laserlichtquelle 28. Die Laserlichtquelle 28 ist bei dem Ausführungsbeispiel der Fig. 2 eine Laserdiode. Die Laserlichtquelle 28 erzeugt einen Laserstrahl, welcher durch eine Optik 27 aufgeweitet wird. Mit 26 ist eine Mittelachse des so aufgeweiteten Laserstrahls bezeichnet.

Diese Mittelachse 26 bildet mit einer Linie 210, welche senkrecht auf dem Glassubstrat 23 steht, einen Beleuchtungswinkel 25. Dieser ist entsprechend einem Beleuchtungswinkel beim Erstellen der holografischen Schicht 24 gewählt. Bei der Beleuchtung wird dann ein virtuelles holografisches Objekt als Fixationstarget 29 erzeugt, welches von einem Auge 21 einer Person entsprechend der Linie 210, welche hier zugleich die Blickrichtung andeutet, betrachtbar ist.

Zu beachten ist, dass die Fig. 2 nicht maßstäblich gezeichnet ist, und insbesondere der Abstand des Fixationstargets 29 von der holografischen Schicht 24 größer sein kann als in der Zeichnung erscheint. Insbesondere ist dieser Abstand größer oder gleich 8 m, so dass die Blickrichtung der Person im Wesentlichen einem Blick ins Unendliche entspricht.

Zudem umfasst die Einrichtung 20 noch eine Glasplatte 22, welche die Einrichtung 20 vor Verschmutzung und auch vor Beschädigung beispielsweise durch versehentliches Anfassen schützt.

Die holografische Schicht 24 der Fig. 2 arbeitet in Transmission, was bedeutet, dass in dem Fall der Fig. 2 die Laserlichtquelle 28 und das Fixationstarget 29 auf der gleichen Seite der holografischen Schicht 24 angeordnet sind, während das Auge 21 auf der anderen Seite angeordnet ist. Das Licht von der Laserlichtquelle geht also durch die holografische Schicht 24 hindurch (Transmission) zu dem Auge 21.

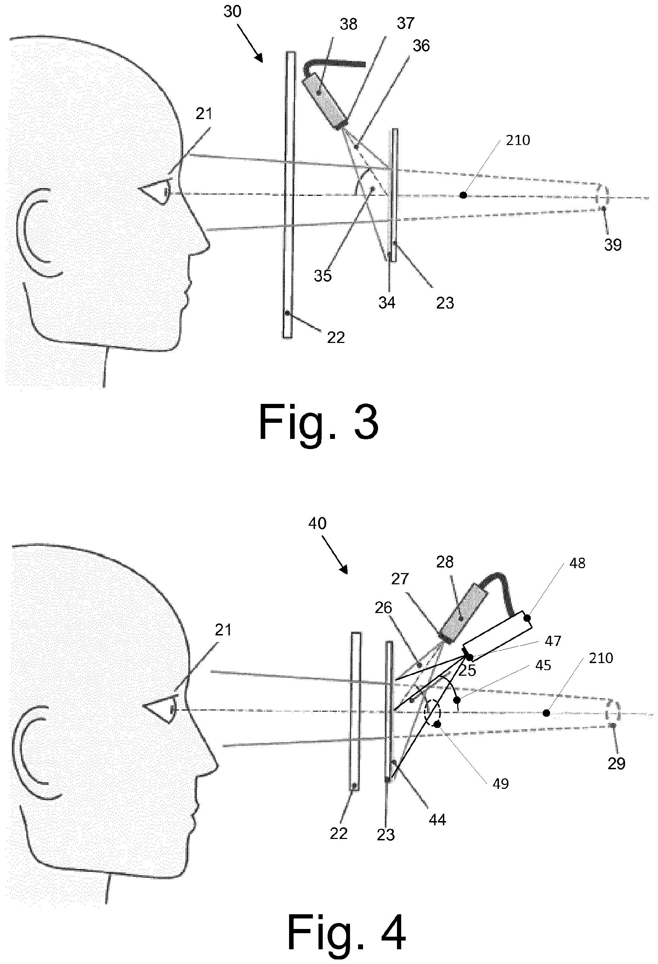

Die Fig. 3 zeigt demgegenüber eine Einrichtung 30, die in Reflexion arbeitet. Elemente der Fig. 3, die Elementen der Fig. 2 entsprechen, tragen die gleichen Bezugszeichen und werden nicht nochmals erläutert. Elemente der Fig. 3, die gegenüber den Elementen der Fig. 2 modifiziert wurden, um eine Anordnung in Reflexion zu erhalten, sind mit dem gleichen Bezugszeichen um 10 erhöht gekennzeichnet.

Bei dem Ausführungsbeispiel der Fig. 3 ist wiederum eine holografische Schicht 34 auf dem Glassubstrat 23 angeordnet. Eine Laserlichtquelle 28 erzeugt einen Laserstrahl, welcher von einer Optik 37 aufgeweitet wird. Mit dem Bezugszeichen 36 ist eine Mittelachse des aufgeweiteten Strahls bezeichnet, welcher einen Winkel 35 zu der senkrechten Linie 210 auf der holografischen Schicht 34 bildet. Der Winkel 35 ist wiederum entsprechend einem Beleuchtungswinkel beim Erstellen des Hologramms gewählt, wie erläutert.

Bei Beleuchtung der holografischen Schicht 34 wird dann ein virtuelles holografisches Objekt als Fixationstarget 39 erzeugt, welches wie das Fixationstarget 29 der Fig. 2 von der Person entsprechend einer Blickrichtung entlang der Linie 210 betrachtet werden kann. Wie die Fig. 2 ist auch die Fig. 3 nicht maßstäblich, und das Fixationstarget 39 weist einen Abstand von 8 m oder mehr von der holografischen Schicht 34 auf.

Die holografische Schicht 34 der Fig. 3 arbeitet wie bereits erwähnt als Reflexionshologramm, das heißt die Lichtquelle 38 beleuchtet die holografische Schicht 34 von einer Seite, von der die holografische Schicht 34 auch mit dem Auge 21 wie dargestellt betrachtet wird, und das Fixationstarget 39 entsteht auf der anderen Seite der holografischen Schicht 34. Das Licht von der Laserlichtquelle 28 wird also in diesem Fall von der holografischen Schicht 34 zu dem Auge 21 reflektiert.

Die Fig. 4 zeigt eine Einrichtung 40 zum wahlweisen Erzeugen zweier Fixationstargets. Die Vorrichtung der Fig. 4 beruht dabei auf der Vorrichtung der Fig. 2, und gleiche Elemente tragen die gleichen Bezugszeichen. Insbesondere wird bei dem Ausführungsbeispiel der Fig. 4 durch Beleuchtung einer holografischen Schicht 44 mit der Laserlichtquelle 28 unter dem Winkel 25 das Fixationstarget 29 erzeugt.

Zudem ist bei dem Ausführungsbeispiel der Fig. 4 eine weitere Laserlichtquelle 48 mit einer entsprechenden weiteren Optik 47 zum Aufweiten des Laserstrahls bereitgestellt, welche eingerichtet ist, die holografische Schicht 44 unter einem Winkel 45 zu beleuchten, der sich von dem Winkel 25 unterscheidet. In der holografischen Schicht 24 sind zwei Hologramme, eines für den Winkel 25 und eines für den Winkel 45, gespeichert, beispielsweise in verschiedenen Schichten eines Volumenhologramms. Bei Beleuchtung der holografischen Schicht 44 mittels der Laserlichtquelle 28 wird wie erläutert das Fixationstarget 29 als Fixationstarget erzeugt. Bei Beleuchtung der holografischen Schicht 44 mit der Laserlichtquelle 48, wird stattdessen ein virtuelles holografisches Objekt als ein Fixationstarget 49 erzeugt, welches näher an dem Auge 21 liegt als das Fixationstarget 29 und wie weiter oben erläutert als Nahblickziel dienen kann. Beispielsweise kann der Abstand des Fixationstargets 49 von der holografischen Schicht 44 zwischen 10 und 50 cm betragen, beispielsweise so, dass der Abstand zu dem Auge 21 ungefähr 30 cm beträgt.

Während das Fixationstarget 49 in der Fig. 4 ebenfalls auf der Linie 210 erzeugt wird, kann es auch abseits dieser Linie 210, beispielsweise nach unten versetzt, erzeugt werden, entsprechend einer Blickrichtung beim Lesen.

In entsprechender Weise, wie mit zwei Lasern 28, 48 in Fig. 4 verschiedene Fixationstargets 29, 49 an verschiedenen Orten erzeugt werden, können zusätzlich oder alternativ auch Fixationstargets mit verschiedenen Farben und/oder Formen wie eingangs erläutert wahlweise erzeugt werden, welche sich dann auch am gleichen Ort befinden können.

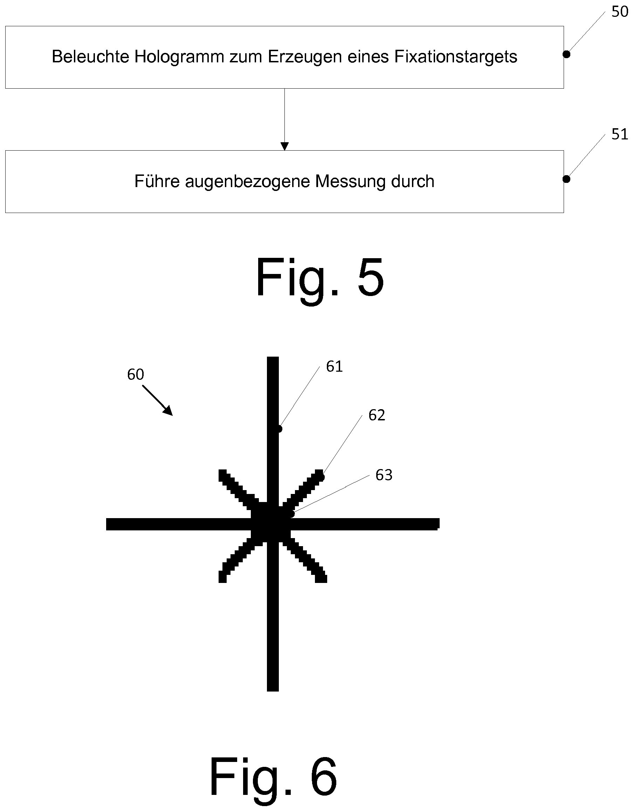

Die Fig. 5 zeigt ein Flussdiagramm eines Verfahrens gemäß einem Ausführungsbeispiel. In Schritt 50 ein Hologramm zum Erzeugen eines Fixationstargets beleuchtet, beispielsweise die holografische Schicht 24 der Fig. 2 zum Erzeugen des Fixationstargets 29, Beleuchten der holografischen Schicht 34 zum Erzeugen des Fixationstargets 39, oder Beleuchten der holografischen Schicht 44 zum Erzeugen des Fixationstargets 29 oder 49.

In Schritt 51 wird dann eine augenbezogene Messung durchgeführt, während eine Person auf das Fixationstarget blickt. Beispielsweise wird eine Zentriermessung mit der Zentriervorrichtung 10 der Fig. 1 oder eine andere der eingangs erwähnten augenbezogenen Messungen durchgeführt.

Die Figur 6 zeigt ein Beispiel für ein Fixationstarget 60, wie es mit den ober erläuterten Vorrichtungen und Verfahren erzeugbar ist. Das Fixationstarget 60 kann dabei als helles (in einer Farbe entsprechend der Farbe der verwendeten Lichtquelle) Fixationstarget vor einem dunklen Hintergrund erzeugt werden.

Das Fixationstarget 60 weist ein größeres Kreuz 61, ein zu dem größeren Kreuz 60 um einen Winkel von 45° verdrehtes kleineres Kreuz 62 sowie einen zentralen Bereich 63, in dem sich Balken der Kreuze 61, 62 schneiden, auf. Die Begriffe "größer" und "kleiner" sind hier relativ zu verstehen, d.h. das größere Kreuz 60 ist wie dargestellt größer als das kleinere Kreuz 62.

Der zentrale Bereich 63 stellt ein Beispiel für einen zu fixierenden Bereich des Fixationstargets 60 dar, d.h. der zu untersuchenden Person wird (durch einen Arzt, Augenoptiker oder auch die Vorrichtung selbst) die Anweisung gegeben, diesen Bereich zu fixieren. Der zentrale Bereich 63 weist dabei wie erläutert eine Ausdehnung entsprechend einem Betrachtungswinkel <1°, insbesondere kleiner 0,5° auf. Der Betrachtungswinkel ist dabei von der Person aus zu sehen und entspricht bei Fixationstargets, die in einem großen Abstand wie den erwähnten 8 m erzeugt werden, näherungsweise einem Betrachtungswinkel von dem Hologramm, das das Fixationstarget erzeugt, aus gesehen.

Das gesamte Fixationstarget 60 weist demgegenüber eine größere Ausdehnung auf, um es stark fehlsichtigen Personen zu ermöglichen, das Fixationstarget zu erkennen. So kann das größere Kreuz 61 eine Ausdehnung entsprechend einem Betrachtungswinkel von 20° aufweisen.

## Patentansprüche

1. Vorrichtung (10) zur Durchführung einer augenbezogenen Messung, umfassend eine Einrichtung (14) zum Erzeugen eines Fixationstargets (29; 39; 49; 60),
wobei die Einrichtung (14) zum Erzeugen des Fixationstargets (29; 39; 49) ein holografisches Element (24; 34; 44) umfasst,
eine Einrichtung (12, 15) zur Durchführung der augenbezogenen Messung, während eine Person auf das Fixationstarget (29; 39; 49; 60) blickt, und eine Beleuchtungseinrichtung (28; 38) zum Beleuchten des holografischen Elements (24; 34; 44), die zum wahlweisen Beleuchten des holografischen Elements (44) mit verschiedenen Beleuchtungsarten, die sich hinsichtlich Lichtwellenlänge, Beleuchtungswinkel und/oder Beleuchtungsort unterscheiden, um zwischen einem Erzeugen eines ersten Fixationstargets (29) und einem Erzeugen eines zweiten Fixationstargets (49) als das Fixationstarget zu wählen, eingerichtet ist, wobei sich das erste Fixationstarget von dem zweiten Fixationstarget hinsichtlich Position und/oder Farbe und/oder Form von dem ersten Fixationstarget unterscheidet, wobei die Vorrichtung eingerichtet ist, das erste Fixationstarget oder das zweite Fixationstarget zum Übermitteln einer über das Vorhandensein des Fixationstargets hinausgehenden Information an die Person auszuwählen, **dadurch gekennzeichnet, dass** die Vorrichtung Kameras (12) umfasst, um eine Position des Kopfes der Person zu erfassen,
und dass die Vorrichtung eingerichtet ist, die Position des Kopfes mit einer Soll-Position zu vergleichen, und dass die Information angibt, ob der Kopf der Person für die Durchführung der augenbezogenen Messung korrekt positioniert ist.

2. Vorrichtung nach Anspruch 1, wobei die Information Hinweise, Anweisungen und/oder Rückmeldungen an die Person umfasst, zusätzlich zum bloßen Vorhandensein des Fixationstargets.

3. Vorrichtung nach einem der Ansprüche 1-2, **dadurch gekennzeichnet, dass** das holografische Element (24; 34; 44) eingerichtet ist, das Fixationstarget (29; 39; 60) in einem Abstand von mindestens 4 m, bevorzugt mindestens 8 m, von dem holografischen Element (24; 34; 44) zu erzeugen.

4. Vorrichtung nach einem der Ansprüche 1-3, **dadurch gekennzeichnet, dass** das holografische Element (24; 34; 44) eingerichtet ist, das Fixationstarget (29; 39; 49; 60) mit zu fixierenden Bereich (63) einer Ausdehnung zu erzeugen, wobei der Arkustangens der Ausdehnung geteilt durch den Abstand des Fixationstargets von dem holografischen Element <1°, bevorzugt <0,5° ist.

5. Vorrichtung nach einem der Ansprüche 1-4, **dadurch gekennzeichnet, dass** die Beleuchtungseinrichtung eine erste Lichtquelle (28) mit einer ersten Lichtwellenlänge und eine zweite Lichtquelle mit einer zweiten Lichtwellenlänge, die sich von einer ersten Lichtwellenlänge unterscheidet, umfasst, wobei das holografische Element eingerichtet ist, das erste Fixationstarget (29; 60) mit einer Farbe entsprechend der ersten Lichtwellenlänge bei Beleuchtung durch die erste Lichtquelle (28) und das zweite Fixationstarget mit einer Farbe entsprechend der zweiten Lichtwellenlänge bei Beleuchtung durch die zweite Lichtquelle zu erzeugen.

6. Vorrichtung nach einem der Ansprüche 1-5, **dadurch gekennzeichnet, dass** die Beleuchtungseinrichtung (28; 38) eingerichtet ist, das holografische Element wahlweise auf eine erste Beleuchtungsart oder eine zweite Beleuchtungsart zu beleuchten, wobei sich die erste Beleuchtungsart von der zweiten Beleuchtungsart hinsichtlich eines Beleuchtungswinkels und/oder hinsichtlich einer Wellenlänge und/oder hinsichtlich eines Beleuchtungsortes unterscheidet,
wobei das holografische Element eingerichtet ist, bei Beleuchtung mit der ersten Beleuchtungsart das erste Fixationstarget (29) mit einer ersten Form zu erzeugen, und eingerichtet ist, bei Beleuchtung mit der zweiten Beleuchtungsart das zweite Fixationstarget (29) mit einer zweiten Form zu erzeugen, die sich von der ersten Form unterscheidet.

7. Vorrichtung nach einem der Ansprüche 1-5, **dadurch gekennzeichnet, dass** die Beleuchtungseinrichtung (28; 38) eingerichtet ist, das holografische Element (24; 34; 44) wahlweise auf eine erste Beleuchtungsart oder eine zweite Beleuchtungsart zu beleuchten, wobei sich die erste Beleuchtungsart von der zweiten Beleuchtungsart hinsichtlich eines Beleuchtungswinkels und/oder hinsichtlich einer Wellenlänge und/oder hinsichtlich eines Beleuchtungsortes unterscheidet, wobei das holografische Element (44) eingerichtet ist, bei Beleuchtung mit der ersten Beleuchtungsart das erste Fixationstarget (29) zu erzeugen und bei Beleuchtung mit der zweiten Beleuchtungsart das zweite Fixationstarget (49) an einer anderen Position zu erzeugen als das erste Fixationstarget (29).

8. Vorrichtung nach Anspruch 7, **dadurch gekennzeichnet, dass** ein Abstand des zweiten Fixationstargets (49) von dem holografischen Element (44) kleiner als 1 m ist.

9. Vorrichtung nach einem der Ansprüche 1-8, **dadurch gekennzeichnet, dass** das holografische Element (24; 34; 44) eingerichtet ist, das Fixationstarget (29) mit einem ersten Teil, der einen Abstand von kleiner 1 m zu dem holografischen Element (24; 34; 44) aufweist, und einem zweiten Teil, der einen Abstand von größer 4 m zu dem holografischen Element (24; 34; 44) aufweist, zu erzeugen.

10. Verfahren zur Durchführung einer augenbezogenen Messung, umfassend:
Beleuchten eines holografischen Elements (24; 34; 44) zum Erzeugen eines Fixationstargets (29; 39; 49; 60), und
Durchführen der augenbezogenen Messung, während eine Person den Blick auf das Fixationstarget richtet, wobei das Beleuchten des Hologramms ein wahlweises Beleuchten des holografischen Elements (44) mit verschiedenen Beleuchtungsarten, die sich hinsichtlich Lichtwellenlänge, Beleuchtungswinkel und/oder Beleuchtungsort unterscheiden, um zwischen einem Erzeugen eines ersten Fixationstargets (29) mittels einer ersten Beleuchtungsart und einem Erzeugen eines zweiten Fixationstargets (49) mittels einer zweiten Beleuchtungsart als das Fixationstarget zu wählen, umfasst, wobei sich das erste Fixationstarget von dem zweiten Fixationstarget hinsichtlich Position, Farbe und/oder Form von dem ersten Fixationstarget unterscheidet, und das wahlweise Beleuchten auf die erste Beleuchtungsart oder die zweite Beleuchtungsart erfolgt, um der Person eine über das Vorhandensein des Fixationstargets hinausgehende Information zu übermitteln, **gekennzeichnet durch** Erfassen einer Position des Kopfes der Person mittels Kameras (12), und Vergleichen der erfassten Position des Kopfes mit einer Soll-Position, wobei die Information angibt, ob der Kopf der Person für die Durchführung der augenbezogenen Messung korrekt positioniert ist.

11. Verfahren nach Anspruch 10, wobei die Information Hinweise, Anweisungen und/oder Rückmeldungen an die Person umfasst, zusätzlich zum bloßen Vorhandensein des Fixationstargets.

## Claims

1. Apparatus (10) for performing an eye-related measurement, comprising a device (14) for generating a fixation target (29; 39; 49; 60), wherein the device (14) for generating the fixation target (29; 39; 49) comprises a holographic element (24; 34; 44),
a device (12, 15) for performing the eye-related measurement while a person is looking at the fixation target (29; 39; 49; 60), and
an illumination device (28; 38) for illuminating the holographic element (24; 34; 44), which is configured for selectively illuminating the holographic element (44) with different illumination types that differ in terms of light wavelength, illumination angle and/or illumination location to select between generating a first fixation target (29) and generating a second fixation target (49) as the fixation target, wherein the first fixation target differs from the second fixation target with respect to position and/or colour and/or shape of the first fixation target, wherein the apparatus is configured to select the first fixation target or the second fixation target for transmitting information that goes beyond the presence of the fixation target to the person,
**characterized in that** the apparatus comprises cameras (12) to capture a position of the head of the person, and **in that** the apparatus is configured to compare the position of the head to an intended position, and **in that** the information indicates whether the head of the person is correctly positioned for the performance of the eye-related measurement.

2. Apparatus according to Claim 1, wherein the information comprises suggestions, instructions and/or feedback for the person in addition to the mere presence of the fixation target.

3. Apparatus according to either of Claims 1 and 2, **characterized in that** the holographic element (24; 34; 44) is configured to generate the fixation target (29; 39; 60) at a distance of at least 4 m, preferably at least 8 m, from the holographic element (24; 34; 44).

4. Apparatus according to one of Claims 1-3, **characterized in that** the holographic element (24; 34; 44) is configured to generate the fixation target (29; 39; 49; 60) with a region (63) of an extent that is to be fixated on, wherein the arctangent of the extent divided by the distance of the fixation target from the holographic element is <1°, preferably <0.5°.

5. Apparatus according to one of Claims 1-4, **characterized in that** the illumination device comprises a first light source (28) having a first light wavelength and a second light source having a second light wavelength differing from a first light wavelength, wherein the holographic element is configured to generate the first fixation target (29; 60) in a colour corresponding to the first light wavelength upon illumination by the first light source (28) and to generate the second fixation target in a colour corresponding to the second light wavelength upon illumination by the second light source.

6. Apparatus according to one of Claims 1-5, **characterized in that** the illumination device (28; 38) is configured to illuminate the holographic element selectively in a first type of illumination operation or a second type of illumination operation, wherein the first type of illumination operation differs from the second type of illumination operation with respect to an illumination angle and/or with respect to a wavelength and/or with respect to an illumination location, wherein the holographic element is configured to generate the first fixation target (29) having a first shape upon illumination with the first type of illumination operation and is configured to generate the second fixation target (29) having a second shape different from the first shape upon illumination with the second type of illumination operation.

7. Apparatus according to one of Claims 1-5, **characterized in that** the illumination device (28; 38) is configured to selectively illuminate the holographic element (24; 34; 44) according to a first type of illumination operation or a second type of illumination operation, wherein the first type of illumination operation differs from the second type of illumination operation with respect to an illumination angle and/or with respect to a wavelength and/or with respect to an illumination location, wherein the holographic element (44) is configured to generate the first fixation target (29) upon illumination with the first type of illumination operation and to generate the second fixation target (49) at a different position than the first fixation target (29) upon illumination with the second type of illumination operation.

8. Apparatus according to Claim 7, **characterized in that** a distance of the second fixation target (49) from the holographic element (44) is less than 1 m.

9. Apparatus according to one of Claims 1-8, **characterized in that** the holographic element (24; 34; 44) is configured to generate the fixation target (29) with a first part having a distance of less than 1 m from the holographic element (24; 34; 44) and a second part having a distance of greater than 4 m from the holographic element (24; 34; 44).

10. Method for performing an eye-related measurement, comprising:
illuminating a holographic element (24; 34; 44) for generating a fixation target (29; 39; 49; 60), and performing the eye-related measurement while a person directs their gaze at the fixation target, wherein illuminating the hologram comprises selectively illuminating the holographic element (44) with different illumination types that differ in terms of light wavelength, illumination angle and/or illumination location to select between generating a first fixation target (29) by way of a first type of illumination operation and generating a second fixation target (49) as the fixation target by means of a second type of illumination operation, wherein the first fixation target differs from the second fixation target with respect to position, colour and/or shape of the first fixation target, and selective illumination according to the first type of illumination operation or the second type of illumination operation takes place to transmit information that goes beyond the presence of the fixation target to the person,
**characterized by** capturing a position of the head of the person using cameras (12) and comparing the captured position of the head to an intended position, wherein the information indicates whether the head of the person is correctly positioned for the performance of the eye-related measurement.

11. Method according to Claim 10, wherein the information comprises suggestions, instructions and/or feedback for the person in addition to the mere presence of the fixation target.

## Revendications

1. Dispositif (10) permettant d'effectuer une mesure relative à l'œil, comprenant un moyen (14) permettant de générer une cible de fixation (29 ; 39 ; 49 ; 60),
dans lequel le moyen (14) permettant de générer la cible de fixation (29 ; 39 ; 49) comprend un élément holographique (24 ; 34 ; 44),
un moyen (12, 15) permettant de réaliser la mesure relative à l'œil pendant qu'une personne regarde la cible de fixation (29 ; 39 ; 49 ; 60), et
un moyen d'éclairage (28 ; 38) permettant d'éclairer l'élément holographique (24 ; 34) ; 44), qui est conçu pour éclairer sélectivement l'élément holographique (44) à l'aide de différents types d'éclairage qui diffèrent en ce qui concerne la longueur d'onde de la lumière, l'angle d'éclairage et/ou l'emplacement d'éclairage, afin de choisir entre la génération d'une première cible de fixation (29) et la génération d'une seconde cible de fixation (49) en tant que cible de fixation, dans lequel la première cible de fixation diffère de la seconde cible de fixation en ce qui concerne la position et/ou la couleur et/ou la forme de la première cible de fixation,
dans lequel
le dispositif est conçu pour sélectionner la première cible de fixation ou la seconde cible de fixation pour transmettre à la personne une information concernant la présence de la cible de fixation,
**caractérisé en ce que** le dispositif comprend des caméras (12) pour détecter une position de la tête de la personne, et **en ce que** le dispositif est conçu pour comparer la position de la tête à une position de consigne, et **en ce que** l'information indique si la tête de la personne est correctement positionnée pour effectuer la mesure relative à l'œil.

2. Dispositif selon la revendication 1, dans lequel l'information comprend des indications, des instructions et/ou un retour d'information destiné à la personne, en plus de la simple présence de la cible de fixation.

3. Dispositif selon l'une des revendications 1-2, **caractérisé en ce que** l'élément holographique (24 ; 34 ; 44) est conçu pour générer la cible de fixation (29 ; 39 ; 60) à une distance d'au moins 4 m, de préférence d'au moins 8 m, de l'élément holographique (24 ; 34 ; 44).

4. Dispositif selon l'une des revendications 1-3, **caractérisé en ce que** l'élément holographique (24 ; 34 ; 44) est conçu pour générer la cible de fixation (29 ; 39 ; 49 ; 60) avec une zone (63) à fixer ayant une dimension, dans lequel l'arctangente de la dimension divisée par la distance de la cible de fixation par rapport à l'élément holographique est <1°, de préférence <0,5°.

5. Dispositif selon l'une des revendications 1-4, **caractérisé en ce que** le dispositif d'éclairage comprend une première source de lumière (28) ayant une première longueur d'onde de lumière et une seconde source de lumière ayant une seconde longueur d'onde de lumière qui diffère d'une première longueur d'onde de lumière, dans lequel l'élément holographique est conçu pour générer la première cible de fixation (29 ; 60) avec une couleur correspondant à la première longueur d'onde de la lumière lors d'un éclairage par la première source de lumière (28) et la seconde cible de fixation avec une couleur correspondant à la seconde longueur d'onde de la lumière lors d'un éclairage par la seconde source de lumière.

6. Dispositif selon l'une des revendications 1 à 5, **caractérisé en ce que** le moyen d'éclairage (28 ; 38) est conçu pour éclairer l'élément holographique sélectivement selon un premier type d'éclairage ou un second type d'éclairage, dans lequel le premier type d'éclairage diffère du second type d'éclairage en ce qui concerne un angle d'éclairage et/ou une longueur d'onde et/ou un emplacement d'éclairage,
dans lequel l'élément holographique est conçu pour générer la première cible de fixation (29) avec une première forme lors d'un éclairage avec le premier type d'éclairage et est conçu pour générer la seconde cible de fixation (29) avec une seconde forme différente de la première forme lors d'un éclairage avec le second type d'éclairage.

7. Dispositif selon l'une des revendications 1 à 5, **caractérisé en ce que** le moyen d'éclairage (28 ; 38) est conçu pour éclairer l'élément holographique (24 ; 34 ; 44) sélectivement dans un premier mode d'éclairage ou un second mode d'éclairage, dans lequel le premier type d'éclairage diffère du second type d'éclairage en ce qui concerne un angle d'éclairage et/ou une longueur d'onde et/ou un emplacement d'éclairage,
dans lequel l'élément holographique (44) est conçu pour générer la première cible de fixation (29) lors d'un éclairage avec le premier type d'éclairage et pour générer la seconde cible de fixation (49) à une position différente de la première cible de fixation (29) lors d'un éclairage avec le second type d'éclairage.

8. Dispositif selon la revendication 7, **caractérisé en ce qu'**une distance de la seconde cible de fixation (49) par rapport à l'élément holographique (44) est inférieure à 1 m.

9. Dispositif selon l'une des revendications 1-8, **caractérisé en ce que** l'élément holographique (24 ; 34 ; 44) est conçu pour générer la cible de fixation (29) à l'aide d'une première partie située à une distance inférieure à 1 m de l'élément holographique (24 ; 34 ; 44) et d'une seconde partie située à une distance supérieure à 4 m de l'élément holographique (24 ; 34 ; 44) .

10. Procédé permettant d'effectuer une mesure relative à l'œil, comprenant :
l'éclairage d'un élément holographique (24 ; 34 ; 44) pour générer une cible de fixation (29 ; 39 ; 49 ; 60), et
la réalisation de la mesure relative à l'œil pendant qu'une personne regarde la cible de fixation, dans lequel l'éclairage de l'hologramme comprend l'éclairage sélectif de l'élément holographique (44) avec différents types d'éclairage qui diffèrent en ce qui concerne la longueur d'onde de la lumière, l'angle d'éclairage et/ou l'emplacement d'éclairage, afin de choisir entre la génération d'une première cible de fixation (29) au moyen d'un premier type d'éclairage et la génération d'une seconde cible de fixation (49) au moyen d'un second type d'éclairage en tant que cible de fixation, dans lequel la première cible de fixation diffère de la seconde cible de fixation en ce qui concerne la position, la couleur et/ou la forme de la première cible de fixation, et l'éclairage sélectif est effectué avec le premier type d'éclairage ou le second type d'éclairage afin de transmettre à la personne une information concernant la présence de la cible de fixation, **caractérisé en outre par**
la détection d'une position de la tête de la personne au moyen de caméras (12), et
la comparaison de la position détectée de la tête à une position de consigne, dans lequel l'information indique si la tête de la personne est correctement positionnée pour effectuer la mesure relative à l'œil.

11. Procédé selon la revendication 10, dans lequel l'information comprend des indications, des instructions et/ou un retour d'information destiné à la personne, en plus de la simple présence de la cible de fixation.
